# EUROPÄISCHE PATENTSCHRIFT

(11) **EP 2 987 400 B1**
(45) Veröffentlichungstag und Bekanntmachung des Hinweises auf die Patenterteilung: **16.10.2019**
(21) Anmeldenummer: 15181693.1
(22) Anmeldetag: 20.08.2015
(51) Int. Cl.: A01G 33/00, C12M 1/24, C12M 1/00, C12N 1/12, C12M 1/02, C12M 1/26

(54) **VERFAHREN ZUM GEWINNEN VON PHYTOPLANKTON**
METHOD FOR THE PRODUCTION OF PHYTOPLANKTON
PROCEDE DE PRODUCTION DE PHYTOPLANCTON

(30) Priorität: 20.08.2014 DE 102014111913
(43) Veröffentlichungstag der Anmeldung: 24.02.2016
(73) Patentinhaber: Agriculture New Energy GmbH, 39387 Oschersleben (DE)
(72) Erfinder: Ihlow, Bernd, 63599 Biebergemünd (DE)
(74) Vertreter: Stoffregen, Hans-Herbert

(56) Entgegenhaltungen:
- EP-A1- 2 316 917

## Beschreibung

Die Erfindung bezieht sich auf ein Verfahren zur Gewinnung von phototrophen Organismen, wie Phytoplankton, insbesondere Mikroalgen, wie Chlorella vulgaris, wobei phototrophe Organismen und ein wässriges Medium in transparente Flaschen gefüllt werden, die zumindest zeitweise einer künstlichen Beleuchtung ausgesetzt werden, wobei der in den Flaschen vorhandenen Substanzen Kohlenstoffquellen und Nährstoffe zugesetzt sind oder werden, die Flaschen während der Kultivierung einer Bewegung ausgesetzt werden und zum Ernten der phototrophen Organismen zumindest ein Teil der in den Flaschen vorhandenen Substanzen aus den Flaschen entfernt und sodann die phototrophen Organismen abgetrennt werden, wobei die Flaschen während des Kultivierens mittels einer Fördereinrichtung an künstlichen Lichtquellen vorbei transportiert werden.

Der DE 10 2012 214 493 A1 ist ein Photobioreaktor zu entnehmen, bei dem ein in Etagen ausgebildetes transparentes Leitungssystem von einer Kultursuspension wie Algensubstrat durchströmt wird, um phototrophe Organismen wie Algen zu kultivieren.

In kleinformatigen Behältnissen mit einem Volumen von vorzugsweise 0,1 bis 10 Litern werden nach der DE 10 2009 028 059 phototrophe Organismen wie Spirulina oder Chlorella kultiviert. Die Behältnisse werden über einen Zeitraum von z. B. 1 bis 4 Wochen gelagert. Zur Vermeidung von Sedimentationen werden die Behältnisse z. B. mittels Rütteln, Schütteln oder Wenden in Bewegung gehalten. Die phototrophen Organismen werden sodann mittels Zentrifugation geerntet.

Der WO 98/13469 A1 sind ein Verfahren und eine Vorrichtung zum Kultivieren von Zellen und Geweben zu entnehmen. Hierzu werden in ein durchscheinendes Behältnis, das die Form eines Schlauchbeutels hat, Zellen- oder Gewebekulturen eingefüllt. Im Bodenbereich ist eine Öffnung vorgesehen, um die Kulturen zuzuführen.

Aus der EP 2 316 917 A1 ist ein Verfahren zur mixotrophen Kultivierung von Mikroorganismen und/oder Zellen bekannt. Hierzu werden die Kulturen auf eine Hohlfaserschicht aufgebracht, die ihrerseits auf einem Transportband vorliegt, das an einer natürlichen und einer künstlichen Lichtquelle vorbeigeführt wird. Am Ende des Transportweges werden die Mikroorganismen bzw. Zellen aufgefangen und sodann weiterverarbeitet.

Bei Photobioreaktoren zeigt sich der Nachteil, dass diese konstruktiv aufwendig gestaltet sind und im Falle einer Kontamination der gesamte Inhalt des Reaktors vernichtet werden muss. Nachteilig bei entsprechenden Bioreaktoren ist auch, dass zum Fördern der die phototrophen Organismen enthaltenen wässrigen Medien, die auch als Nährflüssigkeit bezeichnet werden können, darauf zu achten ist, dass die Organismen nicht zerstört werden. Daher müssen kostenträchtige Pumpen wie Exzenterschneckenpumpen zum Einsatz gelangen.

Bei der batchweisen Gewinnung von phototrophen Organismen sind relativ lange Lagerzeiten erforderlich, wobei zusätzlich mechanische Vorrichtungen benötigt werden, um Ablagerungen, also Sedimentationen, zu vermeiden.

Wünschenswert wäre daher eine Leistungssteigerung zur großtechnischen Gewinnung phototropher Organismen wie Phytoplankton, das im Kosmetikbereich, in der Pharmaindustrie, als Ersatz für Erdöl und bei der Herstellung von Kunststoffen von besonderer Bedeutung ist.

Der vorliegenden Erfindung liegt die Aufgabe zu Grunde, ein Verfahren der eingangs genannten Art so weiterzubilden, dass eine großtechnische Gewinnung von phototrophen Organismen, insbesondere Mikroalgen, möglich ist, ohne dass ein hoher konstruktiver Aufwand erforderlich ist, gleichzeitig jedoch sichergestellt ist, dass etwaige Kontaminationen nur isoliert auftreten können, ohne große Chargen vernichten zu müssen.

Zur Lösung der Aufgabe sieht die Erfindung im Wesentlichen vor, dass die Flaschen während des Transports oder bei einer Transportunterbrechung einer zusätzlichen Zwangsbewegung aus der Gruppe Schütteln, Drehen, Wenden, Rollen ausgesetzt werden und/oder die Substanz in den Flaschen mittels einer Rühreinrichtung gerührt oder durch Einbauten bewegt oder durchmischt wird.

Abweichend von vorbekannten Batchverfahren werden die nachstehend auch als Behältnisse bezeichneten Flaschen nicht gelagert, sondern dem Grunde nach fortwährend oder über längere Zeiträume während des Kultivierens transportiert. Hierdurch erfolgt eine aufgezwungene Bewegung der in den Behältnissen vorhandenen Substanzen, also der Flüssigkeit mit in dieser vorhandenen phototrophen Organismen, so dass Ablagerungen ausgeschlossen sind. Gleichzeitig wirkt während des Transportes künstliches Licht im hinreichenden Umfang auf die phototrophen Mikroorganismen ein, so dass die phototrophen Organismen zur Erzielung einer großen Population und in kurzer Zeit stets mit Licht gewünschter Wellenlängen beaufschlagt werden können.

So ist bei der Süßwasseralge Chlorella vulgaris vorgesehen, dass die Organismen mit Licht im Wellenlängenbereich 430 ± 10 nm und 680 ± 10 nm beaufschlagt werden. Als künstliche Lichtquellen können LEDs, OLEDs aber auch gewöhnliche Leuchtstoffröhren, Glühlampen, Halogenstrahler oder sonstige Lichtquellen benutzt werden, die Licht im erforderlichen Spektralbereich emittieren.

Dabei ist vorgesehen, dass die Behältnisse während des Transports einer zusätzlichen Zwangsbewegung wie Schütteln, Drehen, Wenden ausgesetzt werden, wodurch die Kultivierung der Organismen verstärkt wird.

Ergänzend besteht auch die Möglichkeit, dass die in dem jeweiligen Behältnis vorhandene Substanz mittels einer Rühreinrichtung in Bewegung versetzt wird.

Durch all diese Maßnahmen kann das Kultivieren der phototrophen Organismen verstärkt werden.

In Weiterbildung der Erfindung ist vorgesehen, dass die Behältnisse einer Station oder mehreren Stationen zugeführt, insbesondere durch diese transportiert werden, in der bzw. in denen die Behältnisse bzw. den in diesen vorhandenen Substanzen geeignete Nährmedien zugegeben werden. Diese schließen insbesondere Kohlenstoffquellen ein. Hierbei kann es sich um organische oder anorganische Kohlenstoffquellen, wie Bicarbonate, Natriumhydrogencarbonate oder um CO₂ handeln, das in Tablettenform oder gasförmig zugeführt werden kann. Die Erfindung wird jedoch nicht verlassen, wenn entsprechende Nährsubstanzen und für die Photosynthese erforderliche Stoffe zusammen mit dem Abfüllen der Behältnisse zugeführt werden.

Des Weiteren zeichnet sich die Erfindung dadurch aus, dass die in den jeweiligen Behältnissen vorhandenen Substanzen vorzugsweise während des Transports, ggfs. auch nach Ausschleusen aus dem Transportweg, in Bezug auf zumindest ein Charakteristikum aus der Gruppe pH-Wert, optisch Dichte, Sauerstoffgehalt, elektrische Leitfähigkeit gemessen werden.

Insbesondere durch die Bestimmung der optischen Dichte bzw. des Sauerstoffgehalts wird festgestellt, ob phototrophe Organismen im hinreichenden Umfang entstanden sind, um sodann die Kulturen zu ernten.

Das Ernten erfolgt vorzugsweise derart, dass die Behältnisse weitgehend entleert werden, wobei vorzugsweise 10 % bis 20 % an Substanz in dem Behältnis verbleibt, die als Ausgangssuspension dient, um neue phototrophe Organismen wie Phytoplankton zu kultivieren. Es ist sodann nur erforderlich, wässriges Medium hinzuzufügen. Hierbei kann es sich um das Filtrat handeln, das bei dem Zentrifugieren der den Behältnissen entnommenen Substanzen anfällt, um die phototrophen Organismen von der Flüssigkeit zu trennen. Andere Trennverfahren kommen selbstverständlich gleichfalls in Frage.

Das dem Behältnismittel zugeführte Filtrat kann ggfs. zuvor einer Behandlung wie Entkeimung unterzogen werden.

Anstelle der optischen Dichte oder ergänzend kann auch die Sauerstoff-Konzentration ermittelt werden, um den Grad der Kultivierung zu ermitteln und somit das Ernten einzuleiten; denn die Sauerstoffkonzentration ist auch ein Maß für die stattfindende Photosynthese.

In Abhängigkeit von den Werten der ermittelten Charakteristika können während des Transports - ggfs. nach Ausschleusen von Behältnissen auf dem Transportweg - Nährstoffe zugegeben werden, um im erforderlichen Umfang und in der gewünschten Zeit phototrophe Organismen zu kultivieren.

Insbesondere sieht die Erfindung vor, dass während des Kultivierens die Behältnisse bzw. die in diesen vorhandene Substanzen einer Temperatur T mit Raumtemperatur ≤ T ≤ 30 °C, insbesondere 25 °C ≤ T ≤ 28 °C, ausgesetzt werden. Der pH-Wert sollte auf leicht sauer eingestellt werden, wobei der bevorzugte Wertebereich beträgt 6, 7 ≤ pH < 7 .

Die bei der Fest-Flüssigkeitstrennung angefallenen Feststoffe können schockgefrostet werden. Es besteht jedoch auch die Möglichkeit, die Feststoffe in einem Vakuumtrockenschrank zu trocknen und sodann das entstehende Produkt z. B. in einer Hammermühle zu zerkleinern, um Pulver zu gewinnen, das in Beutel verpackt oder zu Kapseln oder Tabletten verpresst werden kann.

Mit der erfindungsgemäßen Lehre ergibt sich reproduzierbar die Möglichkeit, großtechnisch phototrophe Organismen zu kultivieren, wobei insbesondere Mikroalgen wie Chlorella vulgaris gewonnen werden können.

Das Abfüllen des wässrigen Mediums in die Behältnisse, insbesondere Wasser bzw. Filtrat, kann mit einer einfachen Dosierpumpe, wie Kreiselpumpe, erfolgen, da die Organismen nicht mitgefördert werden.

Um die Substanzen unabhängig von der transportbedingten Bewegung zusätzlich zu durchmischen, besteht eine Vielzahl von Möglichkeiten wie Drehen oder Wenden der Behältnisse während des Transports oder das Fördern über Rütteltische, um nur beispielhaft mechanische Hilfsmittel anzugeben. Dabei besteht auch die Möglichkeit, die Behältnisse über schiefe Ebenen oder in Führungen schwerkraftbedingt rollen zu lassen. Unabhängig hiervon ist jedoch vorgesehen, dass während des Transports oder der zusätzlichen oder alternativ einwirkenden Zwangsbewegungen die Behältnisse der zur Kultivierung erforderlichen Strahlung ausgesetzt sind. Hierzu ist insbesondere vorgesehen, dass die Behältnisse an Lichtleisten entlang geführt werden, die selbst Führungen bilden können.

Insbesondere können in dem Behältnis Schikanen eingesetzt sein, um bei einer Drehbewegung im erforderlichen Umfang eine Durchmischung zu erzielen. Vorzugsweise befinden sich im Transportweg eine oder mehrere Messstationen, um nach Vorliegen der gewünschten Population die Mikroorganismen wie Mikroalgen-Population zu bestimmen.

Ist vorzugsweise im Transportweg eine Messstation vorgesehen, in der den Behältnissen bzw. den in diesen vorhandenen Substanzen CO₂ zugeführt wird, so kann in derselben oder in einer anderen Station entstandenes O₂ gemessen bzw. abgesaugt werden. Ist eine gewünschte Dichte der phototrophen Organismen in einem Behältnis erreicht, wird die Nährstofflösung teilweise entfernt, um in einer weiteren Station die phototrophen Organismen wie Algen von der Nährstofflösung zu trennen, insbesondere durch Zentrifugation. Das Zentrifugat wird ggfs. nach einer Entkeimung erneut verwendet.

Es besteht auch die Möglichkeit, die Produktion unter Reinraum-Bedingungen durchzuführen. Hierzu ist insbesondere vorgesehen, dass der Produktionsraum gegenüber der Umgebung mit Überdruck beaufschlagt ist.

Selbstverständlich können die Behältnisse nach einer festzulegenden Anzahl von Befüllungen vollständig gereinigt werden, um sicherzustellen, dass Kontaminationen unterbleiben.

Die erfindungsgemäß vorgesehene fortwährende Bewegung der in den Behältnissen vorhandenen Substanzen stellt nicht nur sicher, dass Ablagerungen unterbleiben, sondern die gute Durchmischung gewährleistet eine im Vergleich zum Stand der Technik schnellere Kultivierung der phototrophen Organismen, also das Anwachsen insbesondere der Mikroalgenpopulation, da im hinreichenden Umfang eine Beaufschlagung mit der die Photosynthese bewirkender Strahlung erfolgt.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus der nachfolgenden Beschreibung von der Zeichnung zu entnehmenden Ausführungsbeispielen.

Es zeigen:
- Fig. 1: eine Anordnung zur Gewinnung von phototrophen Organismen und
- Fig. 2: Prinzipdarstellungen von Anordnungen, mit denen Behältnisse während ihres Transports einer weiteren Zwangsbewegung ausgesetzt werden.

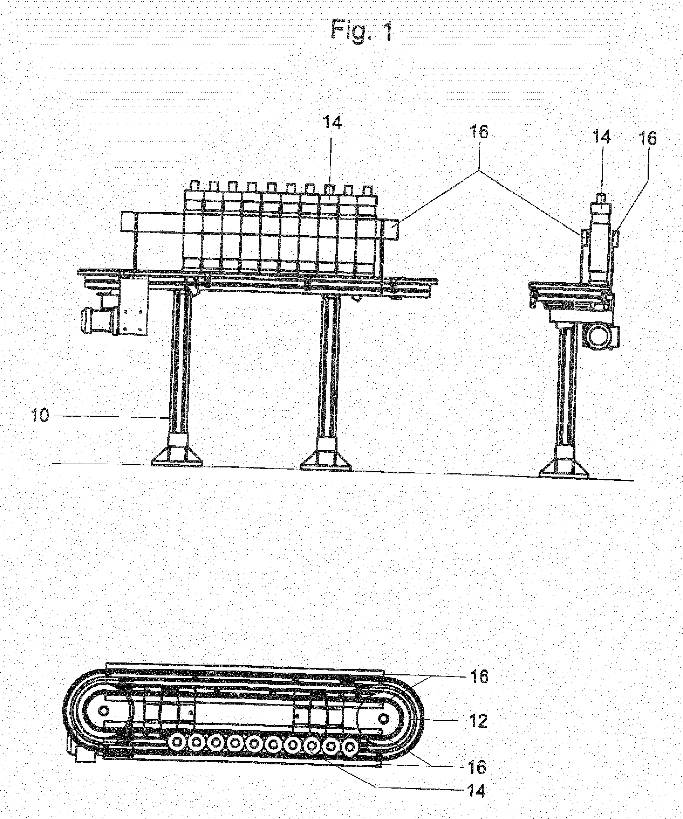

In Fig. 1 ist rein prinzipiell eine Vorrichtung 10 in Seiten-, Vorder- und Draufsicht mit einem umlaufenden Transportband 12 dargestellt, auf dem als Flaschen 14 ausgebildete Behältnisse angeordnet werden, die fortwährend entlang von Lichtleisten 16 gefördert werden, die Strahlen emittieren, um in den Flaschen vorhandene phototrophe Organismen, die sich in einer Nährstofflösung befinden, zu kultivieren. Die Flaschen 14 weisen z. B. ein Fassungsvermögen zwischen 1 Liter und 20 Liter oder sogar mehr auf und sind verschließbar. In den Flaschen 14 können sich Einbauten wie Schikanen befinden, damit während der Bewegung auf dem Transport- bzw. Förderband 12 eine Zwangsmischung der in den Flaschen 14 vorhandenen Substanzen erfolgt, unabhängig von der durch den Transport selbst bedingten Bewegung bzw. Vermischung.

Durch all diese Maßnahmen ist sichergestellt, dass die Substanzen in einem Umfang durchmischt werden, dass die phototropen Organismen im erforderlichen Umfang mit Licht beaufschlagt werden, um eine Kultivierung und damit ein Anwachsen von phototrophen Organismen sicherzustellen.

In der Praxis sind die Transportwege länger dimensioniert als in der Prinzipdarstellung angedeutet, wobei eine Bewegung der Flaschen 14 nicht nur in einer Ebene, sondern dreidimensional erfolgen kann. Dabei besteht die Möglichkeit, dass die Flaschen 14 zum Überwinden unterschiedlicher Niveaus einer Rollbewegung ausgesetzt werden, um ein zusätzliches Durchmischen zu erreichen. Auch während der diesbezüglichen Bewegung werden die Flaschen 14 und damit die phototrophen Organismen mit Strahlung beaufschlagt, die zur Gewinnung von Mikroalgen, insbesondere des Typs Chlorella vulgaris, mit Licht in Wellenlängenbereichen um 430 nm und 680 nm beaufschlagt werden.

Mit anderen Worten sollten die Flaschen 14 und damit die Nährstofflösungen fortwährend dem Licht ausgesetzt sein.

Zu den Flaschen 14 ist anzumerken, dass diese als Synonym für Behältnisse geeigneter Geometrien zu verstehen sind, um eine Nährstofflösung aufzunehmen, deren Inhalt grundsätzlich fortwährend mit einer Strahlung beaufschlagt wird und die Möglichkeit bieten, den Inhalt zu durchmischen.

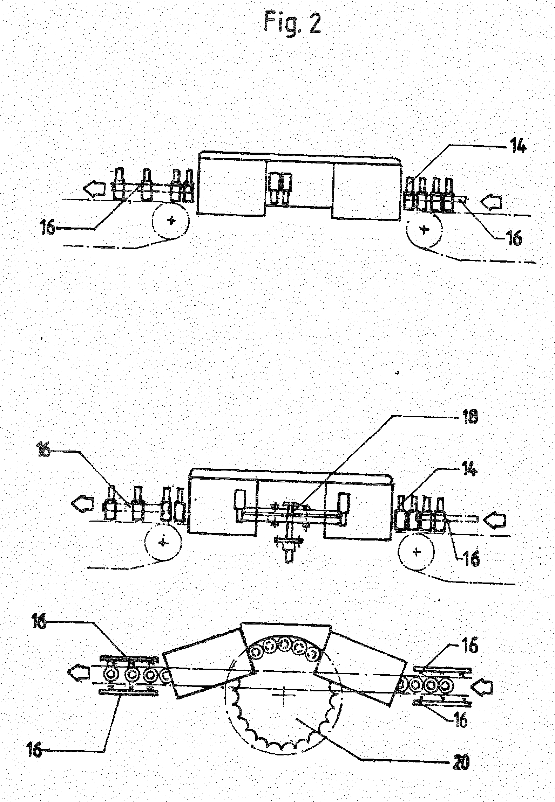

Anhand der Fig. 2 soll verdeutlicht werden, dass die Flaschen 14 während ihres Transportweges zusätzlich gewendet bzw. gedreht werden können. Dies ergibt sich aus den zwei oberen Darstellungen der Fig. 2. Eine Wendeeinrichtung 18 ist auch der mittleren Darstellung zu entnehmen, wobei zusätzlich ein Schütteln erfolgen kann. Bei der untersten Darstellung in Fig. 2 werden die Flaschen 14 an einen Drehtisch 20 übergeben, auf dem die Flaschen während ihres Transports zusätzlich um ihre Längsachsen gedreht werden.

Durch all diese Möglichkeiten ist sichergestellt, dass die in den Flaschen 14 sich in einer Suspension befindenden phototrophen Organismen optimal einer Strahlung ausgesetzt werden, die das Wachstum fördert. Befinden sich in den Behältnissen 14 spezielle Einbauten, die ein Durchmischen bzw. Bewegen dieser Substanz bzw. Suspension in der Flasche 14 ermöglichen, sind grundsätzlich Maßnahmen wie Drehen oder Wenden der Flaschen 14 nicht erforderlich

Um den bei der Photosynthese entstehenden Sauerstoff abzuführen, werden die Flaschen 14 während des Transportes entsprechenden Absaugstationen zugeführt. Dabei besteht auch die Möglichkeit, die Flaschen 14 aus dem Transportweg herauszuschleusen und nach der Sauerstoffentnahme wieder in den Transportweg einzuschleusen. Gleiche Maßnahmen sind möglich, um Charakteristika der Substanz bzw. der Suspension zu ermitteln, die sich in den Flaschen 14 befindet und Rückschlüsse bieten, in welchem Umfang Mikroalgen gewachsen sind. Dabei erfolgt insbesondere eine optische Dichtemessung. Aber auch der Sauerstoffgehalt in der Suspension ist ein Maß für die Photosynthese und somit für die Kultivierung, also dem Wachstum der phototrophen Organismen, wie Mikroalgen.

Um z. B. in 24 Stunden eine Tonne Algen des Typs Chlorella vulgaris herzustellen, werden 50.000 Flaschen mit einem Aufnahmevolumen von 20 Liter pro Flasche benutzt. Bei der Befüllung der Flaschen, also Behältnisse, werden 5 % des eingesetzten flüssigen Mediums wie Wasser mit Algensubstanz versetzt, die aus einer Vorkultur stammen kann. Ggfs. können bei der Befüllung gleichzeitig eine oder mehrere Nährsubstanzen zugegeben sein. Beispielhaft können diese eine oder mehrere aus der Gruppe enthalten: CO₂, N, P, K, Mg, Ca, S, Fe. Die Reihenfolge gibt die Wichtigkeit an, d. h., in der Reihenfolge werden ggfs. Nährstoffe bzw. Zusätze beigegeben. Dies schließt auch die Möglichkeit ein, Kohlendioxid wie CO₂-Tabs mit einzufüllen. Durch die CO₂-Tabs ergibt sich der Vorteil, dass das CO₂ langsam an die Substanz, die im eigentlichen eine Suspension aus Nährstoffen, Wasser und phototrophen Organismen, wie Mikroalgen ist, abgegeben wird. Ein Injizieren während des Wachstums bzw. Kultivierens ist sodann grundsätzlich nicht erforderlich.

Die entsprechend abgefüllten Flaschen bzw. Behältnisse werden sodann mit einer Fördergeschwindigkeit zwischen 0,1 m/s und 0,5 m/s transportiert, wobei während des Transportes das erforderliche Durchmischen mit der Folge gegeben ist, dass die phototrophen Organismen im hinreichenden Umfang der Strahlung ausgesetzt werden, an der die Flaschen bzw. Behältnisse vorbeigeführt werden, um das erforderliche und schnelle Wachstum zu erreichen. Hierzu werden insbesondere Lichtleisten benutzt, die mit LEDs bestückt sind und die Flaschen mit einer Bestrahlungsstärke (Lichtausbeute) von zumindest 100 Lm/W bei 6500 K beaufschlagen. Die LEDs emittieren Strahlen im Wellenlängenbereich von 430 ± 10 nm und 680 ± 10 nm.

Der Abstand zwischen Lichtquelle und Flasche 14 sollte im Bereich zwischen 10 mm und 100 mm liegen.

Selbstverständlich bestünde auch die Möglichkeit, die Flaschen nicht kontinuierlich, sondern zumindest zeitweise im stop-and-go-Betrieb zu transportieren. Letzteres bewirkt eine Bewegung der Suspension in der Flasche.

Sobald mittels der optischen Dichte und/oder dem Sauerstoffgehalt die gewünschte Konzentration an Algen erreicht ist, werden die Flaschen bzw. Behältnisse z. B. mittels eines Roboters entleert, um in einer separaten Station die Algen von der Nährstofflösung zu trennen. Hierzu können Zentrifugen benutzt werden, wobei das anfallende Filtrat - ggfs. nach einer Entkeimung - erneut zum Befüllen von Behältnissen benutzt wird. Die Mikroalgen werden sodann z. B. in einem Vakuumtrockenschrank getrocknet, um anschließend mittels z. B. einer Hammermühle zerkleinert zu werden. Das dabei entstehende Pulver kann in Beutel verpackt oder zu Kapseln oder Tabletten verpresst werden. Alternativ besteht die Möglichkeit eines Schockfrostens.

Beim Entleeren der Flaschen 14 bzw. Behältnisse wird darauf geachtet, dass ca. 10 % bis 20 % an Substanz bzw. Suspension in der Flasche 14 bzw. dem Behältnis verbleibt, die sodann als Ausgangslösung für eine neue Kultivierung nach Befüllen der Flasche 14 bzw. des Behältnisses mit insbesondere Filtrat benutzt wird.

## Patentansprüche

1. Verfahren zur Gewinnung von phototrophen Organismen, wie Phytoplankton, insbesondere Mikroalgen, wie Chlorella vulgaris, wobei phototrophe Organismen und ein wässriges Medium in transparente Flaschen (14) gefüllt werden, die zumindest zeitweise einer künstlichen Beleuchtung (16) ausgesetzt werden, wobei den in den Flaschen vorhandenen Substanzen Kohlenstoffquellen und Nährstoffe zugesetzt sind oder werden, die Flaschen während der Kultivierung der phototrophen Organismen einer Bewegung ausgesetzt werden und zum Ernten der phototrophen Organismen zumindest ein Teil der in den Flaschen vorhandenen Substanzen aus den Flaschen entfernt und sodann die phototrophen Organismen abgetrennt werden, wobei die Flaschen (14) während des Kultivierens mittels einer Fördereinrichtung (12) an künstlichen Lichtquellen (16) vorbei transportiert werden,
**dadurch gekennzeichnet,**
**dass** die Flaschen (14) während des Transports oder bei einer Transportunterbrechung einer zusätzlichen Zwangsbewegung aus der Gruppe Schütteln, Drehen, Wenden, Rollen ausgesetzt werden und/oder die Substanz in den Flaschen mittels einer Rühreinrichtung gerührt oder durch Einbauten bewegt oder durchmischt wird.

2. Verfahren nach Anspruch 1,
**dadurch gekennzeichnet,**
**dass** die Flaschen (14) einer Station oder mehreren Stationen zugeführt, insbesondere durch diese transportiert werden, in der bzw. in denen den Flaschen bzw. der in diesen vorhandenen Substanz eine Kohlenstoffquelle und/oder Nährstoffe zugegeben und/oder aus dem Behältnis Sauerstoff abgezogen wird.

3. Verfahren nach zumindest einem der vorhergehenden Ansprüche,
**dadurch gekennzeichnet,**
**dass** die in der jeweiligen Flasche (14) vorhandene Substanz, vorzugsweise während des Transports, in Bezug auf zumindest ein Charakteristikum aus der Gruppe pH-Wert, optische Dichte, Sauerstoffgehalt, elektrische Leitfähigkeit gemessen wird.

4. Verfahren nach zumindest einem der vorhergehenden Ansprüche,
**dadurch gekennzeichnet,**
**dass** die Flasche (14) volumenmäßig mit mehr als 70%, vorzugsweise 80% bis 90 %, mit der Substanz zur Kultivierung der phototrophen Organismen gefüllt wird.

5. Verfahren nach zumindest einem der vorhergehenden Ansprüche,
**dadurch gekennzeichnet,**
**dass** zum Ernten der phototrophen Organismen aus de Flasche (14) Substanz in einem Umfang entfernt wird, dass von der Substanz 10 % bis 20 % in dem Behältnis verbleibt, die zur erneuten Kultivierung sodann mit wässrigem Medium, das zumindest Wasser enthält, aufgefüllt wird.

6. Verfahren nach zumindest einem der vorhergehenden Ansprüche,
**dadurch gekennzeichnet,**
**dass** beim Ernten anfallendes Filtrat, ggfs. nach einer Behandlung, einer Flasche (14) als das wässrige Medium zugeführt wird.

7. Verfahren nach zumindest einem der vorhergehenden Ansprüche,
**dadurch gekennzeichnet,**
**dass** die in der Flasche (14) vorhandene Substanz während des Kultivierens auf eine Temperatur T mit Raumtemperatur ≤ T ≤ 30 °C, insbesondere 25 °C ≤ T ≤ 28 °C, ausgesetzt wird und/oder auf einen pH-Wert eingestellt wird mit 6,7 ≤ pH<7.

8. Verfahren nach zumindest einem der vorhergehenden Ansprüche,
**dadurch gekennzeichnet ,**
**dass** die beim Ernten von der Flüssigkeit getrennten Feststoffe schockgefrostet oder einem Trockenschrank wie Vakuumtrockenschrank zugeführt werden, dass vorzugsweise das nach dem Trocknen entstandene Produkt, insbesondere mittels einer Hammermühle, zerkleinert wird, und dass so gewonnenes Pulver vorzugsweise in Beutel verpackt oder zu Kapseln oder Tabletten verpresst wird.

9. Verfahren nach zumindest einem der vorhergehenden Ansprüche,
**dadurch gekennzeichnet,**
**dass** die Flaschen (14) an den künstlichen Lichtquellen (16) mit einer Geschwindigkeit v mit 0,1 m/s ≤ v ≤ 0,5 m/s, insbesondere in einem Abstand a mit 10 ≤ mm ≤ a ≤ 100 mm vorbeigeführt werden, wobei vorzugsweise die Lichtausbeute im Bereich von zumindest 100 Lm/W bei 6500 K liegt.

## Claims

1. Method for production of phototrophic organisms such as phytoplankton, in particular microalgae such as Chlorella vulgaris, where phototrophic organisms and an aqueous medium are filled into transparent bottles (14) which are exposed at least at times to artificial lighting (16), where carbon sources and nutrients are added to the substances present in the bottles, said bottles are subjected to a movement during cultivation of the phototrophic organisms, and for harvesting of the phototrophic organisms at least a part of the substances present in the bottles is removed from said bottles and then the phototrophic organisms are separated, the bottles (14) being conveyed past artificial light sources (16) by means of a conveyor device (12) during cultivation,
wherein
the bottles (14) are subjected during conveying or during an interruption of conveying to an additional forced movement from the group shaking, rotating, turning, rolling, and/or the substance in the bottles is agitated by means of an agitating device, or moved or mixed by internal fittings.

2. Method according to claim 1,
wherein
the bottles (14) are supplied to, in particular conveyed through, a station or several stations, in which a carbon source and/or nutrients are added to the bottles or to the substance present therein, and/or oxygen is extracted from the container.

3. Method according to at least one of the preceding claims,
wherein
the substance present in the respective bottle (14) is measured, preferably during conveying, in respect of at least one characteristic from the group pH value, optical density, oxygen content, electrical conductivity.

4. Method according to at least one of the preceding claims,
wherein
the bottle (14) is filled in terms of volume with more than 70 %, preferably 80 % to 90 %, of the substance for cultivation of the phototrophic organisms.

5. Method according to at least one of the preceding claims,
wherein
for harvesting of the phototrophic organisms substance is removed from the bottle (14) to an extent that 10 % to 20% of said substance remains inside the container, which is then topped up with aqueous medium, containing at least water, for renewed cultivation.

6. Method according to at least one of the preceding claims,
wherein
filtrate generated during harvesting, possibly after a treatment, is supplied as the aqueous medium to a bottle (14).

7. Method according to at least one of the preceding claims,
wherein
the substance present in the bottle (14) is exposed during cultivation to a temperature T with room temperature ≤ T ≤ 30 °C, in particular 25 °C ≤ T ≤ 28 °C, and/or is set to a pH value of 6.7 ≤ pH < 7.

8. Method according to at least one of the preceding claims,
wherein
the solids separated from the liquid during harvesting are shock-frosted or supplied to a drying cabinet such as a vacuum drying cabinet, the product resulting after drying is preferably crushed, in particular by means of a hammer mill, and the powder thus obtained is preferably packaged in bags or compacted into capsules or tablets.

9. Method according to at least one of the preceding claims,
wherein
the bottles (14) are moved past the artificial light sources (16) at a velocity v with 0.1 m/s ≤ v ≤ 0.5 m/s, in particular at a distance a of 10 ≤ mm ≤ a ≤ 100 mm, the light yield being preferably in the range of at least 100 Lm/W at 6500 K.

## Revendications

1. Procédé de production d'organismes phototrophes tels que du phytoplancton, notamment des microalgues telle la Chlorella vulgaris, sachant que des organismes phototrophes et un milieu aqueux sont versés dans des flacons transparents (14) qui sont exposés au moins temporairement à un éclairage artificiel (16), sachant que des sources carbonées et des nutriments sont ou seront ajoutés aux substances présentes dans les flacons, que les flacons sont soumis à un mouvement pendant la culture des organismes phototrophes et que pour récolter les organismes phototrophes, au moins une partie des substances présentes dans les flacons est retirée des flacons et qu'ensuite, les organismes phototrophes sont détachés, sachant que pendant la culture, les flacons (14) sont passés devant des sources lumineuses artificielles (16) au moyen d'un dispositif de convoyage. (12).
**caractérisé en ce**
**que** les flacons (14) sont soumis, pendant le transport ou pendant une interruption du transport, à un mouvement forcé supplémentaire du type agitation, rotation, pivotement, roulis et/ou que la substance dans les flacons est agitée au moyen d'un dispositif agitateur ou bougée ou mélangée par des éléments intégrés.

2. Procédé selon la revendication 1,
**caractérisé en ce**
**que** les flacons (14) sont fournis à une ou plusieurs stations, notamment sont transportés par celle(s)-ci, dans la ou lesquelles stations une source carbonée et/ou des nutriments sont ajoutés aux bouteilles ou à la substance présente dans celles-ci, et/ou que de l'oxygène est extrait du récipient.

3. Procédé selon au moins une des revendications précédentes,
**caractérisé en ce**
**que** la substance présente dans le flacon respectif (14) est mesurée, de préférence pendant le transport, pour déterminer au moins une caractéristique du type valeur du pH, densité optique, teneur en oxygène, conductivité électrique.

4. Procédé selon au moins une des revendications précédentes,
**caractérisé en ce**
**que** le flacon (14) est rempli avec la substance destinée à la culture des organismes phototrophes, à un volume de plus de 70 %, de préférence à un volume situé entre 80 % et 90%.

5. Procédé selon au moins une des revendications précédentes,
**caractérisé en ce**
**que** pour récolter du flacon (14) les organismes phototrophes, de la substance est retirée dans une proportion telle que demeure dans le récipient 10 % à 20 % de la substance qui est complétée ensuite par un milieu aqueux contenant au moins de l'eau, pour une nouvelle culture.

6. Procédé selon au moins une des revendications précédentes,
**caractérisé en ce**
**que** le filtrat résultant de la récolte, le cas échéant après un traitement, est ajouté à un flacon (14) comme milieu aqueux.

7. Procédé selon au moins une des revendications précédentes,
**caractérisé en ce**
**que** la substance présente dans le flacon (14) est soumise pendant la culture à une température T telle que température ambiante ≤ T ≤ 30 °C, notamment 25 °C ≤ T ≤ 28 °C, et/ou est réglée à une valeur de pH telle que 6,7 ≤ pH < 7

8. Procédé selon au moins une des revendications précédentes,
**caractérisé en ce**
**que** les matières solides séparées du liquide lors de la récolte sont congelées instantanément ou mises dans une étuve, telle qu'une étuve à vide, que de préférence, le produit obtenu après le séchage est broyé, notamment au moyen d'un broyeur à marteaux, et que la poudre ainsi obtenue est emballée dans des sachets ou comprimée sous forme de capsules ou de comprimés.

9. Procédé selon au moins une des revendications précédentes,
**caractérisé en ce**
**que** les flacons (14) sont passés devant les sources de lumière artificielle (16) à une vitesse v telle que 0,1 m/s ≤ v ≤ 0,5 m/s, notamment à une distance a telle que
10 mm ≤ a ≤ 100 mm, sachant que de préférence, l'efficacité lumineuse se situe dans une plage d'au moins 100 Lm/W à 6 500 K.
